# EUROPÄISCHE PATENTSCHRIFT

(11) **EP 0 695 737 B1**
(45) Veröffentlichungstag und Bekanntmachung des Hinweises auf die Patenterteilung: **18.06.1997**
(21) Anmeldenummer: 95111426.3
(22) Anmeldetag: 20.07.1995
(51) Int. Cl.: C07C 209/62, C07C 211/52

(54) **Verfahren zur Herstellung nitrosubstituierter Arylamine**
Process for the preparation of substituted nitro-arylamines
Procédé pour la préparation de nitrosubstituées arylamines

(30) Priorität: 02.08.1994 DE 4427249
(43) Veröffentlichungstag der Anmeldung: 07.02.1996
(73) Patentinhaber: BAYER AG, 51368 Leverkusen (DE)
(72) Erfinder: Jautelat, Manfred, Dr., D-51399 Burscheid (DE)

(56) Entgegenhaltungen:
- US-A- 5 117 063
- US-A- 5 252 737
- TETRAHEDRON LETTERS, Bd. 31, Nr. 22, 1990 OXFORD GB, Seiten 3217-3220, N.R. AYYANGAR ET AL. 'A novel reaction of acetanilide with nitrobenzene in DMSO.'

## Beschreibung

Die Erfindung betrifft ein Verfahren zur Herstellung von nitrosubstituierten Arylaminen aus Nitroaromaten, Harnstoffen und Sauerstoff in Gegenwart von Basen.

Aromatische Amine sind wichtige Zwischenprodukte für die Herstellung von Farbstoffen, Pflanzenschutzmittel, Pharmazeutika und für die Fotoindustrie.

Technisch bedeutsame Verfahren zur Herstellung von aromatischen Aminen sind die Reduktion von Nitrogruppen in leicht zugänglichen Nitroaromaten oder die Umsetzung von Halogenaromaten mit Ammoniak oder Aminen. Obwohl diese Verfahren im großen Umfange technisch genutzt werden, ergeben sich wegen der mehrstufigen Reaktion oft nicht zufriedenstellende Ausbeuten. Aus der US-A-5117063 ist ein Verfahren zur Herstellung von p-Nitrodiphenylamin aus Nitrobenzol, Anilin und Sauerstoff in Gegenwart von Basen in polaren Lösungsmitteln bekannt. Auch die direkte Aminierung von Nitrobenzol mit Acetanilid in Gegenwart von Basen in DMSO ist bekannt, wobei als Hauptprodukt p-Nitrosodiphenylamin entsteht (Tetrah. Lett. 1990, Bd. 31, n° 22, 3217-3220). Ferner ist ein Verfahren zur Herstellung von N-aliphatischsubstituierten p-Phenylen-diaminen beschrieben, durch Umsetzung von Nitrobenzol mit aliphatischen Aminen in Gegenwart von Base und Protonen-haltigen Substanzen (US-A- 5,252,737). Desweiteren ist ein Verfahren zur Herstellung von p-nitroaromatischen Amiden bekannt, das die Umsetzung von Nitrobenzol mit Amiden in Gegenwart von speziellen Basen, wie Tetraalkylammoniumhydroxiden, in Gegenwart von Protonen-haltigen Stoffen beschreibt (W0-A-9324447). Diese Verfahren erfordern spezielle Bedingungen oder Basen und/oder liefern Ausbeuten, die nicht immer zufriedenstellend sind.

Überraschenderweise wurde nun ein allgemein anwendbares Verfahren zur direkten Aminierung von nitrosubstituierten Aromaten mit Harnstoffen in Gegenwart von einfachen Basen und Sauerstoff gefunden. Die Umsetzungen führen in guten Ausbeuten zu den entsprechenden Aminen.

Gegenstand der Erfindung ist ein Verfahren zur Herstellung aromatischer Amine der Formel 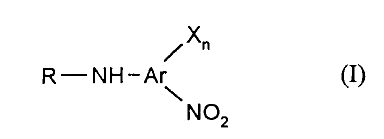 worin
- Ar: einen mono- oder polycyclischen, vorzugsweise mono- oder bicyclischen, aromatischen Rest mit 4 bis 16 C-Atomen, der auch 1 bis 2 Heteroatome aus der Reihe bestehend aus H, O und S enthalten kann,
- R: Wasserstoff, C₁-C₈- Alkyl, C₂-C₈-Alkenyl, C₃-C₇-Cycloalkyl, C₆-C₁₄-Aryl, wobei diese Substituenten 1- bis 3-fach durch Halogen, C₁-C₄-Alkyl, Amino und/oder C₁-C₄-Alkoxy substituiert sein können,
- X: Halogen, Cyano, C₁-C₄-Alkyl, halogeniertes C₁-C₄Alkyl, C₁-C₄-Alkoxy, halogeniertes C₁-C₄-Alkoxy, C₁-C₄-Alkylmercapto, halogeniertes C₁-C₄-Alkylmercapto, C₁-C₄-Alkylsulfonyl oder Nitro,
- n: Null, 1, 2 oder 3, vorzugsweise Null, 1 oder 2, bedeuten,
wobei bei n > 1 die Substituenten X verschieden sein können,
wonach man Nitroaromaten der Formel 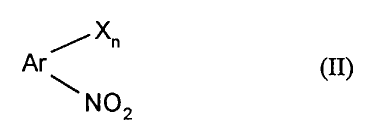 worin
X, Ar und n die oben angegebene Bedeutung haben,
mit Harnstoffen der Formel 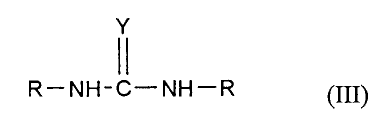 worin die beiden Substituenten R gleich oder verschieden sind und die oben angegebene Bedeutung besitzen und Y für O oder S steht,
in Gegenwart von Basen mit Sauerstoff in polaren Lösungsmitteln umgesetzt werden.

Bevorzugte aromatische C₄-C₁₆-Reste umfassen beispielsweise Benzol-, Naphthalin-, Pyridin-, Chinolin- und Thiophenreste, vorzugsweise Benzol- und Naphthalinreste.

"C₁-C₈-Alkyl" und "C₁-C₄-Alkyl" umfassen lineare und verzweigte Reste wie Methyl, Ethyl, Isopropyl und n-, sek- und tert-Butyl.

"C₂-C₈-Alkenyl" umfaßt Vinyl und Allyl.

"C₃-C₇-Cycloalkyl" umfaßt Cyclopropyl, Cyclopentyl und Cyclohexyl.

"C₆-C₁₄-Aryl" steht für unsubstituierte oder substituierte Arylreste wie Phenyl oder Naphthyl, die einfach oder mehrfach durch Halogen, Alkyl, Nitro, Amino, Alkoxy, Alkylthio, Sulfosäure, Hydroxy, Formyl, Benzoyl, Carboxyl, Cyano, Phenyl und Phenylalkyl substituiert sein können.

"Halogen" steht für Brom, Iod, vorzugsweise Fluor und Chlor.

"Halogeniertes C₁-C₄-Alkyl" umfaßt z. B. Trifluormethyl und Dichlorfluormethyl.

"C₁-C₄-Alkoxy" bedeutet vorzugsweise Methoxy, "halogeniertes C₁-C₄-Alkoxy" steht vorzugsweise für Trifluormethoxy.

"C₁-C₄-Alkylmercapto" bedeutet vorzugsweise Methylmercapto; "halogeniertes C₁-C₄-Alkylmercapto" steht vorzugsweise für Trifluormethylmercapto.

"C₁-C₄-Alkylsulfonyl" steht vorzugsweise für Methylsulfonyl.

Bevorzugte Nitroaromaten (II) umfassen beispielsweise Nitrobenzol, m-Chlornitrobenzol, m-Nitrobenzonitril, m-Trifluormethylnitrobenzol, 3-Fluor-nitrobenzol, 3-Nitrotoluol, 3-Trifluormethoxynitrobenzol, 3-Trifluormethylthio-nitrobenzol, 3,5-Dichlornitrobenzol, 2-Nitrobenzonitril, 2-Nitrobenzoesäure, 1-Nitronaphthalin, 2-Nitronaphthalin, 2-Nitrothiophen, 3-Nitrothiophen, 2-Nitrofuran, N-alkylierte und N-arylierte 2- und 3-Nitropyrrole, 2-, 3- und 4-Nitro-pyridin, 4-Ethoxy-3-nitropyridin, 5-, 6- und 8-Nitrochinolin.

Bevorzugte Harnstoffe der Formel (III) umfassen beispielsweise Harnstoff, Thioharnstoff, Methylharnstoff, N,N'-Dimethylharnstoff, N,N'-Diethylharnstoff, N,N'-Dibuthylthioharnstoff, Phenylharnstoff, N,N'-Diphenylharnstoff, N,N'-Diisopropylthioharnstoff, Allylthioharnstoff N,N'-Di-p-tolylthioharnstoff, N,N'-Di-(4-chlorphenyl)-harnstoff. Besonders bevorzugt sind die symmetrisch substituierten Harnstoffe der Formel (III).

Geeignete Basen sind sowohl organische wie anorganische Basen, bevorzugt werden anorganischen Basen, wie z. B. Alkalihydroxide, Alkaliamide, Alkalialkoxide oder Alkalihydride. Besonders bevorzugt sind Alkalihydroxide, wie z. B. Lithiumhydroxid, Natriumhydroxid, Kaliumhydroxid, Caesiumhydroxid, Kalium-tert.-butoxid. Vorzugsweise setzt man die Basen in Form von Pulvern oder Mikrogranulat (Mikropills) ein.

Sauerstoff kann als reines Gas oder besonders bevorzugt in Gemischen mit anderen Gasen, beispielsweise in Form von Luft, eingesetzt werden.

Geeignete Lösungsmittel für die Herstellung der erfindungsgemäßen Verbindungen (I) umfassen organische und anorganische Lösungsmittel. Bevorzugte organische Lösungsmittel sind polare aprotische, wie beispielsweise Dimethylsulfoxid, Dimethylformamid, N-Methylpyrrolidon, Pyridin, Dioxan, THF, Acetonitril, Sulfolan und deren Gemische. Ein bevorzugtes anorganisches Lösungsmittel ist beispielsweise flüssiger Ammoniak. Geringe Mengen, d. h. bis zu 10 Gew.-%, bezogen auf gesamtes Lösungsmittel, an Protonen-haltigen Lösungsmitteln, wie etwa Wasser, sind akzeptabel. Das bevorzugte Lösungsmittel ist Dimethylsulfoxid.

Die Reaktion kann innerhalb eines weiten Temperaturbereichs durchgeführt werden. Im allgemeinen arbeitet man bei Temperaturen zwischen -35°C und 120°C, vorzugsweise zwischen 20°C und 80°C.

Bei der Durchführung des erfindungsgemäßen Verfahrens arbeitet man in allgemeinen unter Normaldruck; es ist aber auch möglich unter erhöhtem oder vermindertem Druck zu arbeiten.

Bei der Durchführung des erfindungsgemäßen Verfahrens setzt man auf 1 mol des Nitroaromaten der Formel (II) in allgemeinen 0,5 bis 10 mol, vorzugsweise 0,7 bis 2 mol an Harnstoffen der Formel (III) sowie 1 bis 10 Äquivalente, vorzugsweise 2 bis 6 Äquivalente Base ein. Sauerstoff wird vorzugsweise unverdünnt oder verdünnt im Überschuß eingeleitet.

Verwendet man 3-Nitrobenzotrifluorid, N,N'-Diphenylharnstoff und Luft als Ausgangssstoffe und Natriumhydroxid als Base, so kann der Verlauf des Verfahrens zur Herstellung von nitrosubstituierten Aminen durch das folgende Formelschema wiedergegeben werden:

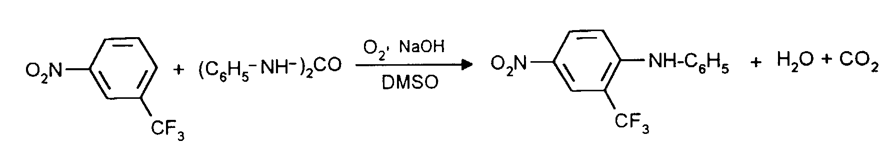

Die Ausgangsstoffe der Formeln (II) und (III) sind bekannt oder können nach bekannten Verfahren hergestellt werden.

Die Aufarbeitung kann nach den üblichen Methoden erfolgen. In allgemeinen verfährt man in der Weise, daß man das Reaktionsgemisch mit Wasser stark verdünnt und das sich abscheidende Reaktionsprodukt abtrennt und isoliert, oder man verdünnt mit Wasser und extrahiert mit einem wenig mit Wasser mischbaren organischen Lösungsmittel. Aus der organischen Phase isoliert man das Produkt, nachdem diese getrocknet und eingeengt wurde.

### Beispiele

### Beispiel 1

### N-Methyl- 4-nitro-2-trifluormethylanilin

1,91 g (10 mmol) 3-Nitrobenzotrifluorid und 1,76 g (20 mmol) N,N'-Dimethylharnstoff werden mit 1,2 g (30 mmol) Natriumhydroxid in Form von Mikroperlen in 30 ml DMSO unter Durchleiten eines Luftstrom 4 h auf 50°C erhitzt. Dann wird mit Ethylacetat verdünnt und mit gesättigter Sodalösung mehrfach ausgeschüttelt. Nach Trocknen der organischen Phase über Natriumsulfat und Abziehen des Lösemittels erhält man 2,2 g (10 mmol, 100 %) N-Methyl- 4-nitro-2-trifluormethylanilin als 95%-iges Produkt vom Fp. 99 - 101°C. Nach Umkristallisation aus Ethanol hat das Produkt einen Fp. von 111 - 112°C.

### Beispiel 2

### N-Butyl-4-nitro-2-trifluormethylanilin

1,91 g (10 mmol) 3-Nitrobenzotrifluorid und 3,76 g (20 mmol) N,N'-Dibutylthioharnstoff werden mit 1,2 g (30 mmol) NaOH-Mikropills in 30 ml abs. DMSO unter Durchleiten von getrockneter Luft 6 h auf 50°C erhitzt. Die Aufarbeitung mit Ethylacetat und Sodalösung führt zu 5,6 g Rohprodukt, das durch Chromatographie mit Petrolether/Ethylacetat (1 : 1 Vol-Teile) über Kiesegel gereinigt wird: 2,1 g (8 mmol, 80 %) N-Butyl-4-nitro-2-trifluormethylanilin als Öl.
- ¹H-NMR (CDCl₃, 200 MHz):: δ 1,0 (t, 3H), 1,5 (m, 2H), 1,7 (m, 2H), 3,3 (m, 2H), 5,05 (s, NH), 6,75 (d, 1H), 8,25 (dd, 1H), 8,4 (d, 1H)

### Beispiel 3

### 4-Nitrodiphenylamin

1,23 g (10 mmol) Nitrobenzol, 2,12 g (10 mmol) N,N'-Diphenylharnstoff und 1,2 g (30 mmol) NaOH-Mikropills werden in 30 ml abs. DMSO unter Durchleiten eines Luftstroms 23 h auf 50°C erhitzt. Nach Aufarbeitung mit Ethylacetat und Sodalösung erhält man 2,0 g (9,3 mmol, 93 %) Produkt, das nach Umkristallisation aus Cyclohexan bei 128°C schmilzt.

Entsprechend den Beispielen 1 bis 3 werden folgende Verbindungen hergestellt:

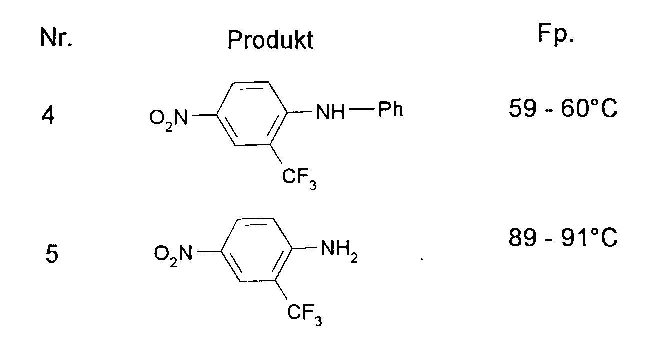

## Patentansprüche

1. Verfahren zur Herstellung von Verbindungen der Formel 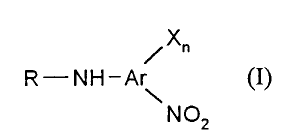 worin
Ar einen mono- oder polycyclischen aromatischen C₄-C₁₆ -Rest, der auch 1 bis 2 Heteroatome aus der Reihe bestehend aus N, O und S enthalten kann,
R Wasserstoff, C₁-C₈-Alkyl, C₂-C₈-Alkenyl, C₃-C₇-Cycloalkyl und C₆-C₁₄-Aryl, wobei diese Substituenten 1- bis 3-fach durch Halogen, C₁-C₄-Alkyl, Amino und/oder C₁-C₄-Alkoxy substituiert sein können,
X Halogen, Cyano, C₁-C₄-Alkyl, halogeniertes C₁-C₄-Alkyl, C₁-C₄-Alkoxy, halogeniertes C₁-C₄-Alkoxy, C₁-C₄-Alkylmercapto, halogeniertes C₁-C₄-Alkylmercapto, C₁-C₄-Alkylsulfonyl oder Nitro,
n Null, 1, 2 oder 3 bedeuten, wobei bei n > 1 die Substituenten X verschieden sein können,
wonach man Nitroaromaten der Formel 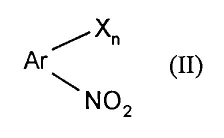 worin
X, Ar und n die oben angegebene Bedeutung haben,
mit Harnstoffen der Formel 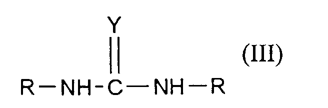 worin die beiden Substituenten R gleich oder verschieden sind und die oben angegebene Bedeutung besitzen und
Y für O oder S steht,
in Gegenwart von Basen mit Sauerstoff in polaren Lösungsmitteln umsetzt.

## Claims

1. Process for the preparation of compounds of the formula 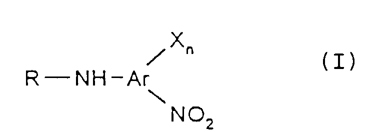 in which
Ar denotes a mono- or polycyclic aromatic C₄-C₁₆ radical which may also contain 1 to 2 heteroatoms from the series comprising N, O and S,
R denotes hydrogen, C₁-C₈ alkyl, C₂-C₈ alkenyl, C₃-C₇ cycloalkyl and C₆-C₁₄ aryl, wherein the latter substituents may be substituted 1 to 3 times with halogen, C₁-C₄ alkyl, amino and/or C₁-C₄ alkoxy,
X denotes halogen, cyano, C₁-C₄ alkyl, halogenated C₁-C₄ alkyl, C₁-C₄ alkoxy, halogenated C₁-C₄ alkoxy, C₁-C₄ alkylmercapto, halogenated C₁-C₄ alkylmercapto, C₁-C₄ alkylsulphonyl or nitro,
n denotes zero, 1, 2 or 3, wherein when n > 1 the substituents X may be different,
by which, in the presence of bases and with oxygen, nitroaromatics of the formula 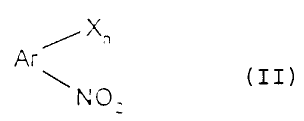 in which
X, Ar and n denote the same as above,
are reacted in polar solvents
with ureas of the formula 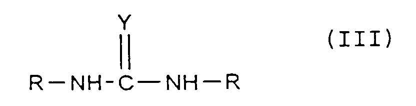 wherein the two substituents R are the same or different and denote the same as above and
Y stands for O or S.

## Revendications

1. Procédé de préparation des composés de formule : 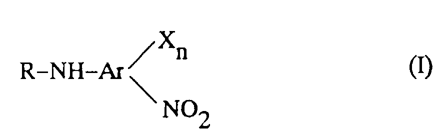 dans laquelle
Ar représente un radical mono- ou polycyclique aromatique en C₄-C₁₆ qui peut également contenir 1 ou 2 hétéroatomes choisis parmi N, O et S,
R représente l'hydrogène, un groupe alkyle en C₁-C₈, alcényle en C₂-C₈, cycloalkyle en C₃-C₇ ou aryle en C₆-C₁₄, ces groupes pouvant eux-mêmes porter 1 à 3 substituants halogéno, alkyle en C₁-C₄, amino et/ou alcoxy en C₁-C₄,
X représente un halogène, un groupe cyano, alkyle en C₁-C₄, alkyle en C₁-C₄ halogéné, alcoxy en C₁-C₄, alcoxy en C₁-C₄ halogéné, alkylmercapto en C₁-C₄, alkylmercapto en C₁-C₄ halogéné, alkylsulfonyle en C₁-C₄ ou nitro,
n est égal à 0, 1, 2 ou 3, les substituants X pouvant être différents lorsque n est supérieur à 1,
selon lequel on fait réagir des dérivés aromatiques nitrés de formule : 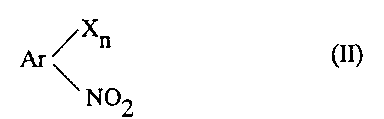 dans laquelle
X, Ar et n ont les significations indiquées ci-dessus, avec des urées de formule : 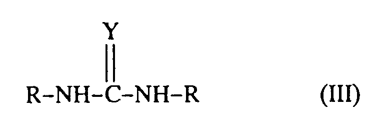 dans laquelle les deux symboles R peuvent avoir des significations identiques ou différentes parmi celles indiquées ci-dessus, et
Y représente O ou S,
en présence de bases et avec l'oxygène dans des solvants polaires.
